# EUROPÄISCHE PATENTSCHRIFT

(11) **EP 0 817 622 B1**
(45) Veröffentlichungstag und Bekanntmachung des Hinweises auf die Patenterteilung: **28.11.2001**
(21) Anmeldenummer: 96911973.4
(22) Anmeldetag: 29.03.1996
(51) Int. Cl.: A61K 9/70, A61K 38/55

(54) **TRANSDERMAL APPLIZIERBARES ARZNEIMITTEL MIT ACE-HEMMERN**
MEDICAMENT WITH ANGIOTENSIN CONVERTING ENZYME (ACE) INHIBITORS SUITABLE FOR TRANSDERMAL APPLICATION
MEDICAMENT A ADMINISTRATION TRANSDERMIQUE, COMPORTANT DES INHIBITEURS DE L'ENZYME DE CONVERSION DE L'ANGIOTENSINE

(30) Priorität: 31.03.1995 DE 19512181
(43) Veröffentlichungstag der Anmeldung: 14.01.1998
(73) Patentinhaber: HEXAL AG, D-83607 Holzkirchen (DE)
(72) Erfinder: FISCHER, Wilfried, 83607 Holzkirchen (DE); KLOKKERS, Karin, 83607 Holzkirchen (DE); SENDL-LANG, Anna, 83607 Holzkirchen (DE)
(74) Vertreter: Boeters, Hans Dietrich, Dr.
(86) Internationale Anmeldenummer: EP9601402
(87) Internationale Veröffentlichungsnummer: WO9629999

(56) Entgegenhaltungen:
- EP-A- 0 425 837
- EP-A- 0 439 430
- WO-A-93/23019

## Beschreibung

Die Langzeittherapie der Hypertonie mit Angiotensin Converting Enzyme-Hemmern (ACE-Hemmern) nimmt einen immer breiteren Raum ein. ACE-Hemmer sind bei guter Verträglichkeit für ihre zuverlässige Wirksamkeit bekannt. Die erste Substanz aus der Klasse der ACE-Hemmer, das Captopril, ist eine sehr hydrophile Substanz, die in unveränderter Form wirksam ist. Die orale Bioverfügbarkeit des Captoprils beträgt etwa 70 %. Neuere ACE-Hemmer, wie Enalapril, werden aus ihrer Vorstufe bei der Leberpassage in die wirksame Komponente Enalaprilat, das heißt die Säureform, metabolisiert. Wie Enalapril sind die ACE-Hemmer Ramipril, Cilacapril, Trandolapril, Benazepril oder Fosinopril lipophile Prodrugs der eigentlichen Wirkform der Dicarbonsäure. Durch die Veresterung jeweils einer Carboxylgruppe des jeweiligen ACE-Hemmers wird die Substanz lipophiler und dadurch für die orale Resorption günstiger. Die orale Bioverfügbarkeit dieser Prodrugs liegt jedoch immer niedriger als die des Captoprils. Sie beträgt beispielsweise für das Benazepril 28 % und für das Trandolapril ca. 40 bis 60 %. Nun ist es eine bekannte Tatsache, daß Substanzen mit geringer Bioverfügbarkeit sehr abhängig von der jeweiligen Metabolisierungsfähigkeit der Patienten sind. Das bedeutet, daß die resultierenden Plasmaspiegel einer sehr hohen Variation unterliegen. Die hohe Variation der Blutspiegel von ACE-Hemmern oder deren Wirkformen führt jedoch zu nicht kalkulierbaren Wirkungsverläufen. Um die Wirkung von ACE-Hemmern nun unabhängig von der metabolischen Lage der Patienten zu machen, wäre eine Arzneiform, die eine zuverlässige, reproduzierbare systemische Zufuhr der Wirkstoffe ermöglicht, wünschenswert. Die transdermale Applikation von Wirkstoffen führt zu einer Umgehung des hepatischen First-Pass-Metabolismus und damit zu einer Ausschaltung der Metabolisierungsvariationen der Leber. Gelänge es nun, ACE-Hemmer in Form ihrer Prodrugs oder Wirkformen transdermal systemisch verfügbar zu machen, könnte eine zuverlässigere gleichmäßige Wirkung erzielbar sein.

Aus WO-A1-9 323 019 ist bereits ein transdermales Reservoir-System mit einem Gehalt an einem ACE-Hemmer und
(a) einer undurchlässigen Abdeckschicht (Backing Layer),
(b) einem schichtartigen Element mit Hohlraum,
(c) einem die Wirkstoffabgabe steuernden Mittel (claim 1) und
(e) einer abziehbaren Deckschicht (Release Liner) auf Papierbasis (Seite 12 Zeilen 7/8) bekannt.

Transdermale Systeme mit einem Gehalt an einem ACE-Hemmer werden ferner in EP-A2-0 439 430 (Reservoir-TTS) und EP-A2-0 468 875 (Matrix-TTS) beschrieben, wobei nach EP-A2-0 468 875 Silikon-Elastomere als Matrixmaterial verwendet werden.

EP-A3-0 452 837 beschreibt ein transdermales System mit einer Matrix auf Basis von u. a. Polyisobutylenkautschuk und mit einem Gehalt an u. a. ACE-Inhibitoren, nämlich Captopril, Enalaprilmaleat, Delapril-Hydrochlorid, Alacepril und (R)-3-[(S)-1-Carboxy-5-(4-piperidyl)-pentyl]-amino-4-oxo-2,3,4,5-tetrahydro-1,5-benzothiazepin-5-Essigsäure. Ramipril und Tandolapril werden nicht genannt. Neben Polyisobutylen-Kautschuken werden als weitere mögliche Matrices auch Polymere vom Acryl-Typ und Silikon-Kautschuke angeführt. Es bestand nun ein Bedürfnis, auch diesen Stand der Technik zu verbessern.

Aufgabe der vorliegenden Erfindung ist es, ein System für die transdermale Zufuhr von ACE-Hemmern vorzusehen, und zwar von Ramipril und/oder Trandolapril, das gegenüber dem Stand der Technik verbessert ist. Insbesondere ist es Aufgabe der, Erfindung, ein System für die transdermale Zufuhr von derartigen ACE-Hemmern vorzusehen, mit dem sich eine Wirksamkeit von bis zu etwa einer Woche erreichen läßt, so daß für etwa eine Woche eine kontinuierliche Abgabe an Wirkstoff und ein therapeutisch wirksamer Plasmaspiegel erreicht werden können, beispielsweise von mehr als 0,5 ng Trandolapril/ml.

Dazu wird erfindungsgemäß ein transdermales System mit einer Matrix auf Basis von Polyisobutylen oder Butylkautschuk und mit einem Gehalt an Ramipril und/oder Trandolapril als ACE-Hemmer vorgesehen. Erfindungsgemäß wurde überraschenderweise festgestellt, daß lipophile ACE-Hemmer oder deren Wirkformen, die die menschliche Haut nur schwer permeieren können, mit Hilfe eines transdermal applizierbaren Arzneimittels mit einer Polyisobutylenmatrix oder Butylkautschukmatrix die Haut gut durchdringen können und einen zuverlässigen, kontinuierlichen Blutspiegel erzeugen.

Erfindungsgemäß kann eine Abgaberate des Wirkstoffs aus beispielsweise einer Polymermatrix von 0,01 bis 0,1 mg Wirkstoff/cm² 24 h und insbesondere 0,025 bis 0,050 mg Wirkstoff/cm² 24 h erreicht werden, so daß ein erfindungsgemäßes transdermales System eine Plasmakonzentration an Wirkstoff in einer therapeutisch wirksamen Menge bietet. Beispielsweise läßt sich für Trandolapril eine therapeutisch wirksame Konzentration im Blut von mehr als etwa 0,5 ng/ml erzielen.

Der Fachmann ist mit geeigneten Matrizes aus Polyisobutylen oder Butylkautschuk vertraut; vgl. beispielsweise Higgins et al. in Satas, Handbook of Pressure Sensitive Adhesive Technology, 14 : 374 etc., Butyl Rubber and Polyisobutylene; Van Nostrand Reinhold, New York.

Bei dem erfindungsgemäßen transdermalen System kann der ACE-Hemmer in einer Konzentration von mindestens 5 Gew.-% und insbesondere in einer Konzentration von 10 bis 20 Gew.-% (bezogen auf die Matrix) vorliegen.

Der ACE-Hemmer kann dabei als Prodrug oder als Wirkform eingesetzt werden.

Die ACE-Hemmer Ramipril und/oder Trandolapril können als deren Wirkformen (Säureformen) sowie deren therapeutisch wirksame Salze vorliegen.

Das erfindungsgemäße transdermale System kann einem Permeationsförderer umfassen, beispielsweise 2-Octyldodecanol.

Bei den erfindungsgemäßen Transdermalsystemen können unterschiedliche Formen Anwendung finden, beispielsweise membran- oder matrixkontrollierte Systeme.

Die microporöse oder semipermeable Membran kann aus einem inerten Polymeren, beispielsweise Polypropylen, Polyvinylacetat oder Silikon bestehen.

Sofern eine Membran vorgesehen ist, kann sie je nach Porenweite eine die Freisetzung des Wirkstoffs kontrollierende Wirkung oder auch keinen Einfluß auf die Wirkstofffreisetzung aus dem System haben.

Gemäß einer speziellen Ausführungsform kann das erfindungsgemäße transdermale System durch
(a) eine undurchlässige Abdeckschicht (Backing Foil),
(b) eine Matrixschicht für den Wirkstoff,
(c) (sofern die Schicht gemäß (b) nicht selbstklebend ist) eine wirkstoffdurchlässige Haftklebeschicht und
(d) gegebenenfalls eine abziebare Deckschicht (Release Liner) gekennzeichnet sein.

Als Matrix kann erfindungsgemäß ein selbstklebender Polyisobutylenkleber verwendet werden.

Bei dem erfindungsgemäßen transdermalen System kann die Abdeckschicht (Backing Foil) aus Polyester, Polypropylen, Polyethylen oder Polyurethan gebildet sein.

Ferner kann bei dem erfindungsgemäßen transdermalen System die abziehbare Deckschicht (Release Liner) aus Polyester, Polypropylen oder beschichtetem Papier (Papier mit Beschichtung) gebildet sein, insbesondere mit einer Silikon- und/oder Polyethylen-Beschichtung.

Schließlich kann das erfindungsgemäße transdermale System eine Abdeckschicht (Backing Foil) und/oder abziehbare Deckschicht (Release Liner) mit einer Dicke im Bereich von 5 bis 100 µm aufweisen.

Nachstehend wird die Erfindung durch Beispiele näher erläutert.

### Beispiele 1 bis 5

Es wird ein transdermales therapeutisches System (TTS) vom Matrixtyp vorgesehen, das beispielsweise durch die folgende Zusammensetzung gekennzeichnet ist.

| | | |
|---|---|---|
| Matrix | Polyisobutylenkleber (MA24® von Adhesive Research Inc., Glen Rock, Pennsylvania, USA) | |
| Abdeckfolie | Polyesterfolie (Hostaphan® RN 19) | |
| Abziehfolie | Polyesterfolie (Gelroflex® PET 75 µm 1-S) oder | |
| | beschichtete Papierfolie (Gelrolease® 603/100 DRS) | |
| Matrixbestandteile | Trandolapril | 10 Gew.-% |
| | 2-Octyldodecanol | 5 Gew.-% |
| | Polyisobutylenkleber (Trockenmasse) | 85 Gew.-% |

### Vergleichsbeispiel 1

Hier wird anstatt eines Polyisobutylenklebers ein Silikonkleber (BIO PSA® X7 4302) verwendet.

Die erhaltenen Ergebnisse sind der folgenden Tabelle zu entnehmen.

Eine Gegenüberstellung von Beispiel 1 und Vergleichsbeispiel 1 zeigt, daß die Wirkstoffaubgabe bei dem erfindungsgemäßen System über einen Zeitraum von 20 Tagen konstant bleibt, während sie beim Vergleichsbeispiel 1 drastisch abfällt.

### Anwendungsbeispiel 1

In einer in-vivo-Vergleichsstudie eines erfindungsgemäßen TTS mit einer oralen Gabe von Trandolapril (Kapsel) wurde bei 6 gesunden Probanden das pharmakokinetische Verhalten für TTS-Applikation geprüft. Dabei wurden im offenen 2-Perioden-cross-over-design die TTS über einen Zeitraum von 7 Tagen (1 TTS 4 Tage, anschließend 1 TTS 3 Tage) appliziert und im Vergleich 7 Tage Lang 1 Kapsel à 2 mg Trandolapril täglich appliziert. Blutproben wurden nach folgenden Zeiten genommen: -0,5; 0; 2; 4; 6; 8; 10; 12; 24; 48; 72; 96; 98; 100; 102; 104; 106; 108; 120; 132; 144; 156; 168 h nach Applikation.

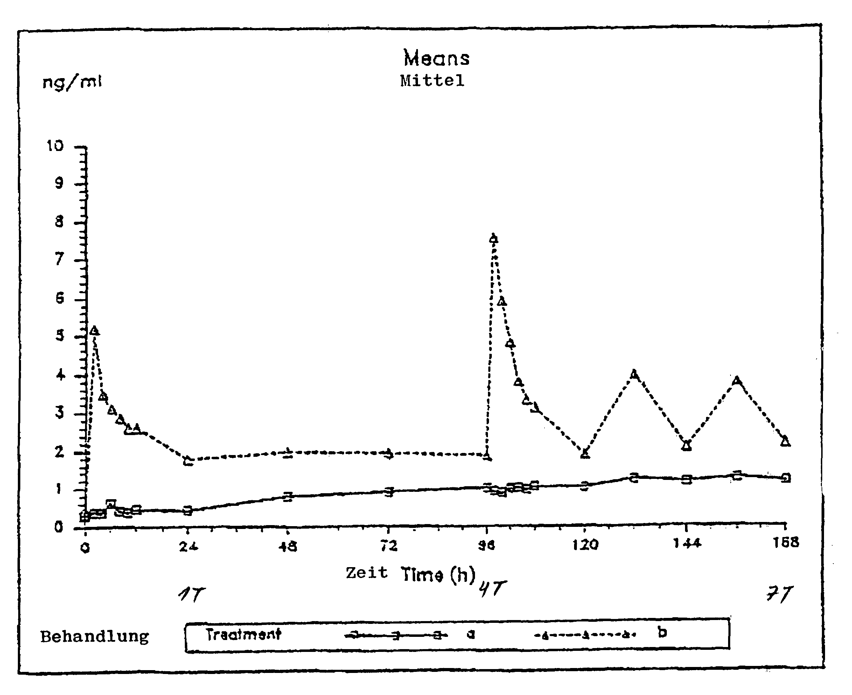

Die pharmakokinetischen Ergebnisse zeigen, daß das TTS ein grundsätzlich anderes Blutspiegelprofil aufweist als die Kapseln. Im Gegensatz zur Kapsel wird ein über den jeweiligen Applikationszeitraum von 3 bzw. 4 Tagen konstanter Blutspiegel erzielt, was therapeutisch auch wünschenswert ist. Nach oraler Gabe steigt die Blutkonzentration schnell an, und zwar werden innerhalb 2 h ca. 5 ng/ml erreicht. Die Elimination erfolgt mit einer Halbwertzeit von ca. 24 h. Im Vergleich dazu ist der Blutspiegelverlauf nach TTS-Gabe gleichmäßiger. In den ersten 4 Tagen der Anwendung ist ein gleichmäßiger leichter Anstieg des Blutspiegels von ca. 0,3 ng/ml nach 6 h auf ca. 1 ng/ml nach 96 h zu beobachten. Nach Wechsel des TTS nach 96 h steigen die Blutspiegel in der zweiten Applikationsperiode nur noch unwesentlich an (**Figur 1**). Damit kommt das Blutspiegelprofil nach Gabe der TTS dem therapeutischen Ideal von konstanten Blutspiegeln während der Behandlung sehr nahe. Unerwünschte Blutspiegelspitzen, die mit unerwünschten Nebenwirkungen wie plötzlichem Blutdruckabfall verbunden sein können, werden sicher vermieden.

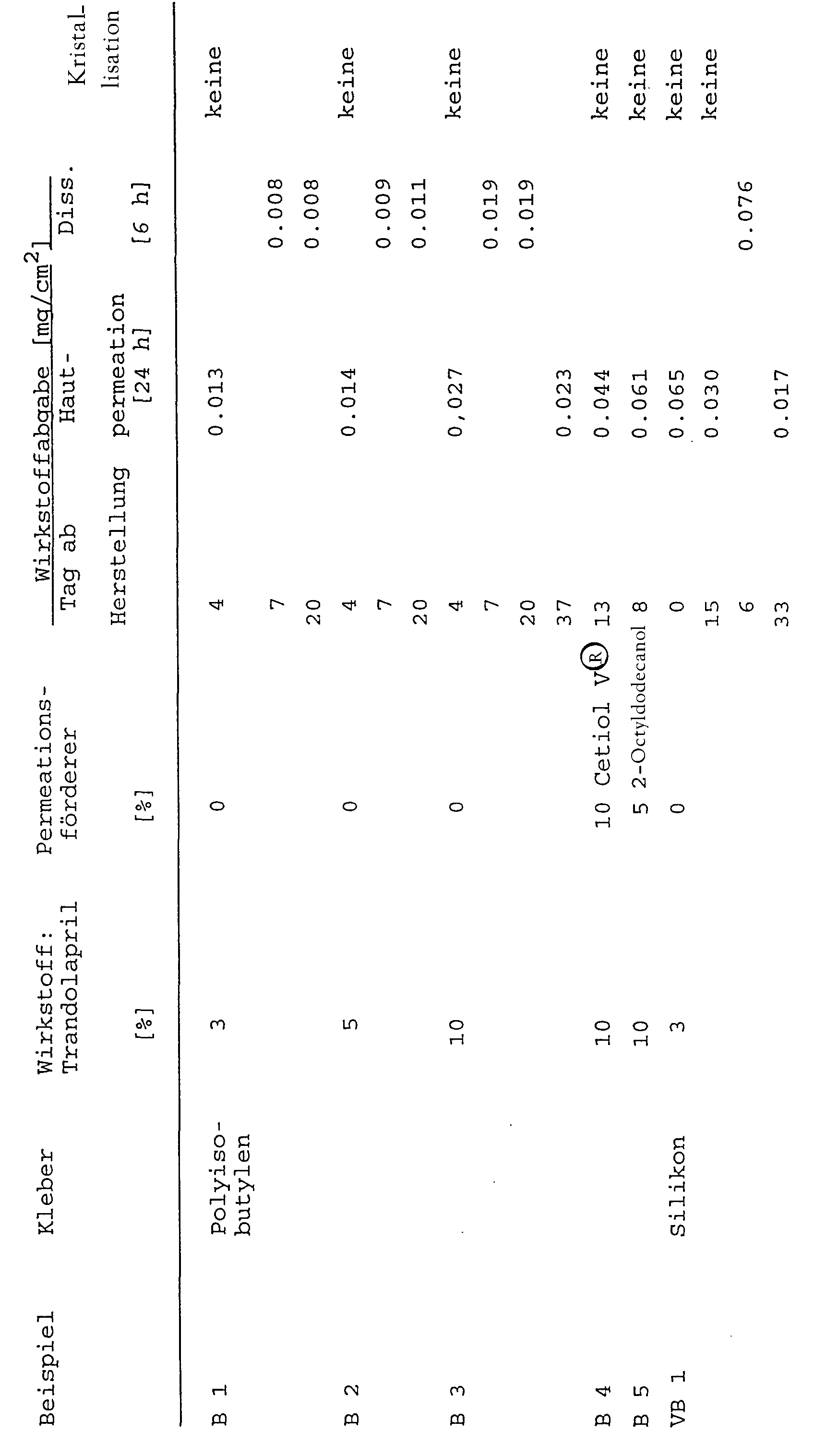

## Patentansprüche

1. Transdermales System mit einer Matrix auf Basis von Polyisobutylen oder Butylkautschuk und mit einem Gehalt an Ramipril und/oder Trandolapril als Angiotensin Converting Enzyme-Hemmer (ACE-Hemmer).

2. Transdermales System nach Anspruch 1, **dadurch gekennzeichnet, daß** der ACE-Hemmer in einer Konzentration von mindestens 5 Gew.-% und insbesondere in einer Konzentration von 10 bis 20 Gew.-% (bezogen auf die Matrix) vorliegt.

3. Transdermales System nach Anspruch 1 oder 2, **dadurch gekennzeichnet, daß** der ACE-Hemmer als Prodrug oder als Wirkform vorliegt.

4. Transdermales System nach einem der vorhergehenden Ansprüche, **dadurch gekennzeichnet, daß** Ramipril und/oder Trandolapril als Wirkform (Säureform) und/oder als therapeutisch wirksames Salz vorliegen.

5. Transdermales System nach einem der vorhergehenden Ansprüche, **dadurch gekennzeichnet, daß** das transdermale System einen Permeationsförderer umfaßt, insbesondere 2-Octyldodecanol.

6. Transdermales System nach einem der vorhergehenden Ansprüche, **dadurch gekennzeichnet, daß** das transdermale System ein Matrix-kontrolliertes oder ein Membran-kontrolliertes System ist.

7. Transdermales System nach Anspruch 6, **dadurch gekennzeichnet, daß** die Membran aus einem inerten Polymeren besteht, insbesondere Polypropylen, Polyvinylacetat oder Silikon.

8. Transdermales System nach einem der Ansprüche 1 bis 5, **gekennzeichnet durch**
(a) eine undurchlässige Abdecksicht (Backing Foil),
(b) eine Matrixschicht für den Wirkstoff,
(c) (sofern die Schicht gemäß (b) nicht selbstklebend ist) eine wirkstoffdurchlässige Haftklebeschicht und
(d) gegebenenfalls eine abziehbare Deckschicht (Release Liner).

9. Transdermales System nach Anspruch 8, **gekennzeichnet durch** einen selbstklebenden Polyisobutylenkleber als Matrix.

10. Transdermales System nach einem der Ansprüche 7 bis 9, **dadurch gekennzeichnet, daß** die Abdeckschicht (Backing Foil) aus Polyester, Polypropylen, Polyethylen oder Polyurethan gebildet ist.

11. Transdermales System nach einem der Ansprüche 7 bis 10, **dadurch gekennzeichnet, daß** die abziehbare Deckschicht (Release Liner) aus Polyester, Polypropylen oder beschichtetem Papier (Papier mit Beschichtung) gebildet ist, insbesondere mit einer Silikon- und/oder Polyethylen-Beschichtung.

12. Transdermales System nach einem der Ansprüche 10 oder 11, **gekennzeichnet durch** eine Abdeckschicht (Backing Foil) und/oder abziehbare Deckschicht (Release Liner) mit einer Dicke im Bereich von 5 bis 100 um.

## Claims

1. Transdermal system having a matrix based on polyisobutylene or butyl rubber and containing ramipril and/or trandolapril as angiotensin-converting enzyme inhibitor (ACE inhibitor).

2. Transdermal system according to Claim 1, **characterized in that** the ACE inhibitor is present in a concentration of at least 5 % by weight and in particular in a concentration of from 10 to 20 % by weight (based on the matrix).

3. Transdermal system according to claim 1 or 2, **characterized in that** the ACE inhibitor is present as a produg or as an active form.

4. Transdermal system according to one of the preceding claims, **characterized in that** ramipril and/or trandolapril are provided as active form (acid form) and/or therapeutically active salt.

5. Transdermal system according to one of the preceding claims, **characterized in that** the transdermal system includes a permeation promoter, in particular 2-octyldodecanol.

6. Transdermal system according to one of the preceding claims, **characterized in that** the transdermal system is a system controlled by a matrix or a membrane.

7. Transdermal system according to Claim 6, **characterized in that** the membrane consists of an inert polymer, in particular polypropylene, polyvinyl acetate or silicone.

8. Transdermal system according to one of Claims 1 to 5, **characterized by**
(a) an impermeable covering layer (backing foil),
(b) a matrix layer for the active compound,
(c) (if the layer according to (b) is not self-adhesive) an active compound-permeable adhesive layer and
(d) if appropriate a covering layer (release liner) which can be torn off.

9. Transdermal system according to Claim 8, **characterized by** a self-adhesive polyisobutylene rubber adhesive as matrix.

10. Transdermal system according to one of Claims 7 to 9, **characterized in that** the covering layer (backing foil) is formed from polyester, polypropylene, polyethylene or polyurethane.

11. Transdermal system according to one of Claims 7 to 10, **characterized in that** the covering layer (release liner) which can be torn off is formed from polyester, polypropylene or coated paper (paper having a coating), in particular with a silicone and/or polyethylene coating.

12. Transdermal system according to one of claims 10 or 11, **characterized by** a covering layer (backing foil) and/or covering layer (release liner) which can be torn off having a thickness in the range from 5 to 100 µm.

## Revendications

1. Système transdermique avec une matrice à base de polyisobutylène ou de caoutchouc butylique et ayant une teneur en Ramipril et/ou en Trandolapril en tant qu'inhi-biteur de l'enzyme de conversion de l'angiotensine (inhi-biteur ECA).

2. Système transdermique selon la revendication 1, **caractérisé en ce que** l'inhibiteur ECA est présent à une concentration d'au moins 5 % en poids et en particulier à une concentration de 10 à 20 % en poids (par rapport à la matrice).

3. Système transdermique selon la revendication 1 ou 2, **caractérisé en ce que** l'inhibiteur ECA est présent en tant que promédicament ou sous une forme active.

4. Système transdermique selon l'une quelconque des revendications précédentes, **caractérisé en ce que** Ramipril et/ou Trandolapril sont présents sous une forme active (forme acide) et/ou en tant que sel actif sur le plan thérapeutique.

5. Système transdermique selon l'une quelconque des revendications précédentes, **caractérisé en ce que** le système transdermique comporte un agent favorisant la perméation, en particulier le 2-octyldodécanol.

6. Système transdermique selon l'une quelconque des revendications précédentes, **caractérisé en ce que** le système transdermique est un système contrôlé par une matrice ou contrôlé par une membrane.

7. Système transdermique selon la revendication 6, **caractérisé en ce que** la membrane est constituée d'un polymère inerte, en particulier le polypropylène, le poly-(acétate de vinyle) ou la silicone.

8. Système transdermique selon l'une quelconque des revendications 1 à 5, **caractérisé par**
(a) une couche de recouvrement imperméable ("backing foil"),
(b) une couche de matrice pour la substance active,
(c) (dans la mesure où la couche selon (b) n'est pas auto-adhésive) une couche adhésive de contact, perméable aux substances actives et
(d) éventuellement une couche de recouvrement que l'on peut enlever ("release liner").

9. Système transdermique selon la revendication 8, **caractérisé par** une colle de polyisobutylène auto-adhésive en tant que matrice.

10. Système transdermique selon l'une quelconque des revendications 7 à 9, **caractérisé en ce que** la couche de recouvrement ("backing foil") est formée de polyester, de polypropylène, de polyéthylène ou de polyuréthanne.

11. Système transdermique selon l'une quelconque des revendications 7 à 10, **caractérisé en ce que** la couche de recouvrement que l'on peut enlever ("release liner") est formée de polyester, de polypropylène ou de papier revêtu (papier avec revêtement), en particulier avec un revêtement de silicone et/ou de polyéthylène.

12. Système transdermique selon l'une quelconque des revendications 10 ou 11, **caractérisé par** une couche de recouvrement ("backing foil") et/ou couche de recouvrement que l'on peut enlever ("release liner") ayant une épaisseur dans une plage de 5 à 100 µm.
